# FASCICULE DE BREVET EUROPEEN

(11) **EP 1 219 587 B1**
(45) Date de publication et mention de la délivrance du brevet: **04.10.2006**
(21) Numéro de dépôt: 01403269.2
(22) Date de dépôt: 17.12.2001
(51) Int. Cl.: C07C 67/08, C07C 69/54

(54) **Procédé de fabrication d'esters carboxyliques insaturés**
Verfahren zur Herstellung von Estern ungesättigter Carbonsäuren
Process for the production of esters of unsaturated carboxylic acids

(30) Priorité: 26.12.2000 FR 0017023
(43) Date de publication de la demande: 03.07.2002
(73) Titulaire: Arkema France, 92800 Puteaux (FR)
(72) Inventeur: Paul, Jean-Michel, 57070 Metz (FR); Busca, Patrick, 57800 Bening les Saint-Avold (FR)
(74) Mandataire: Rieux, Michel

(56) Documents cités:
- FR-A- 2 186 457
- US-A- 3 776 947
- US-A- 5 645 696

## Description

La présente invention porte sur un procédé perfectionné de fabrication d'esters carboxyliques insaturés.

Plusieurs procédés sont décrits dans la littérature pour la synthèse d'esters carboxyliques insaturés.

Les catalyseurs généralement utilisés sont des acides du type acide sulfurique, acide paratoluènesulfonique, acide méthanesulfonique ou similaires. L'eau de réaction est éliminée sous forme d'azéotrope avec l'alcool estérifiant ou avec un solvant comme le cyclohexane, le toluène, etc.

L'utilisation de ces catalyseurs entraîne la formation de sous-produits et nécessite l'utilisation de matériaux adaptés résistant à la corrosion, en particulier avec l'acide sulfurique. L'acide résiduel est ensuite neutralisé. Les opérations de neutralisation/lavages sont lourdes et polluantes.

L'utilisation de résines cationiques fortes de type sulfonique qui ne présentent pas ces désavantages est largement décrite dans la littérature, en matière d'estérification.

Le brevet américain US-A-4 833 267 décrit la synthèse d'esters (méth)acryliques en C₁-C₁₂ en continu dans un réacteur agité à l'aide d'un agitateur mécanique fonctionnant à une puissance de 0,05 à 2kW/m³. La résine cationique est maintenue en suspension dans le milieu réactionnel. Le défaut de cette technique est la contrainte mécanique à laquelle est soumise la résine qui entraîne une fragilisation et la casse de celle-ci avec baisse de l'activité catalytique.

La demande de brevet japonais JP-A-58 192 851 décrit quant à elle l'utilisation d'un réacteur catalytique tubulaire chauffé par une double enveloppe, garni de résine, surmonté d'une colonne à distiller pour l'élimination de l'eau de réaction. Un défaut de cette technique est le contact prolongé de l'eau avec les réactifs et produits, lequel engendre la formation de sous-produits lourds au détriment de la sélectivité.

Le brevet américain US-A-5 645 696 décrit l'utilisation d'un lit catalytique multiétagé alimenté par le haut pour la synthèse d'esters carboxyliques insaturés. Le réacteur est composé de 1 à 10 étages de résine et est surmonté d'une colonne à distiller où s'effectue l'élimination de l'eau de réaction. La vaporisation de l'eau est effectuée à l'aide d'un échangeur externe. Le principal défaut de ce système est sa lourdeur et les risques de bouchage des plateaux par des polymères.

Conformément à la présente invention, il est proposé un procédé amélioré de synthèse en continu d'esters d'acides carboxyliques insaturés par estérification sur résine d'alcools aliphatiques, qui pallie les défauts des procédés revendiqués dans les brevets américains US-A-4 833 267 (bris des résines sous l'effet de l'agitation mécanique), US-A-5 645 696 (complexité de la zone réactionnelle constituée d'un lit multiétagé avec risque d'encrassement par des polymères), et dans la demande japonaise JP-A-58 192 851.

Selon la présente invention, en associant un premier lit fixe de résine alimenté par le bas, avec un réacteur constitué d'une cuve de brassage muni d'une recirculation par le bas au travers d'une cartouche de résine, on obtient des taux de conversion élevés associés à des sélectivités également élevées, ce qui simplifie les opérations de purification en aval. Par ailleurs, les opérations de chargement/déchargement des résines sont extrêmement simples.

La présente invention a donc pour objet un procédé de fabrication d' un ester d'acide acrylique ou méthacrylique par estérification de l'acide acrylique ou méthacrylique par un alcool aliphatique, primaire ou secondaire, en C₁-C₁₂, en présence d'une résine cationique comme catalyseur, l'eau de réaction étant éliminée sous la forme d'un azéotrope avec l'alcool estérifiant ou avec un solvant, caractérisé par le fait que l'on conduit la réaction d'estérification en faisant passer de manière ascendante le mélange des réactifs à travers un lit de ladite résine cationique, dans une boucle de recirculation qui est associée à une cuve agitée dans laquelle est assuré le brassage des réactifs et à partir de laquelle est assurée l'élimination azéotropique de l'eau de réaction.

Le passage sur la résine s'effectue dans le sens ascendant afin de maintenir la résine en suspension et de faciliter les échanges thermiques.

Conformément à un mode de réalisation intéressant de la présente invention, on conduit une réaction partielle en amont de la cuve agitée en adressant le mélange des réactifs de manière ascendante à travers un second lit de la résine cationique comme catalyseur.

L'alcool mis en jeu, est, notamment le n-butanol, le 2-éthylhexanol ou le n-octanol.

Le rapport molaire global Acide/ Alcool est généralement compris entre 0,6 et 1, étant, de préférence, entre 0,7 et 0,9.

On conduit la réaction généralement en présence d'au moins un inhibiteur de polymérisation choisi notamment parmi la phénothiazine, l'hydroquinone, l'éther monométhylique de l'hydroquinone et les phénols encombrés, tels que le 2,6-diterbutylparacrésol, à des teneurs dans le milieu comprises entre 500 et 5000 ppm. Un bullage d'air est généralement effectué dans la cuve de brassage afin de renforcer l'action du ou des inhibiteurs de polymérisation. Ceux-ci sont généralement introduits avec les réactifs et en tête de la colonne à distiller surmontant la cuve agitée.

Conformément à un mode de réalisation particulier du procédé de l'invention, on introduit une partie de l'alcool en tête de la colonne à distiller surmontant la cuve agitée, un ou des inhibiteurs de polymérisation étant avantageusement associés à l'alcool introduit en tête de colonne, la proportion massique de l'alcool ainsi introduit en tête de colonne par rapport à l'alcool introduit dans la cuve 1 étant de 20 à 60 %, de préférence de 30 à 50 %.

La température en tête de lit de résine est généralement comprise entre 70 et 100°C, plus particulièrement entre 80 et 95°C (au-delà de 100°C, on observe une dégradation thermique des résines) ; la température de la cuve de brassage est généralement comprise entre 100 et 110°C, le mélange réactionnel sortant de la cuve de brassage étant refroidi à 80-90°C, puis recirculé au travers du lit de résine.

La résine cationique est avantageusement une résine cationique forte styrène/divinyl benzène à groupements sulfoniques ayant une capacité ionique comprise entre 0,5 et 2,2 équivalents/litre. A titre d'exemples de résines, on peut citer celle commercialisée sous la dénomination "DIAION® PK 208" par la Société "MITSUBISHI CHEM.", et celles commercialisées sous les dénominations "XE 586", "XE 386" et "AMBERLIST 39" par la Société "ROHM & HAAS".

On fait généralement circuler le mélange réactionnel à travers le (premier) lit de résine, le temps de séjour global sur le (premier) lit de résine étant généralement compris entre 0,5 et 2 h.

L'eau générée par l'estérification est distillée sous forme d'azéotrope. L'utilisation d'un solvant aliphatique ou aromatique qui forme un azéotrope avec l'eau : cyclohexane, heptane, toluène, etc... est possible, comme déjà indiqué. Cependant, en règle générale, c'est l'alcool lui-même qui joue ce rôle.

Les vapeurs qui distillent dans la colonne contiennent, outre l'eau, de l'alcool estérifiant, de l'ester carboxylique et de l'acide carboxylique. Un reflux d'alcool contenant 500 à 1000 ppm d'inhibiteurs tels que décrits précédemment permet de limiter la montée d'acide en tête et les pertes via la phase aqueuse de décantation.

Les vapeurs condensées en tête de colonne sont décantées à température ambiante :
- la phase organique est renvoyée dans la colonne à distiller en étant régulièrement déconcentrée en octènes ; et
- la phase aqueuse est éliminée.

La pression de service en tête de la colonne à distiller surmontant la cuve agitée est généralement de 9,33 x 10³ à 5,3 x 10⁴ Pa (70 à 400 mm Hg).

Dans le mode de réalisation préféré précité suivant lequel un premier étage de résine est placé en amont, la température en tête du second lit est généralement de 80 à 95°C, et l'on fait fonctionner le réacteur contenant le second lit à la pression atmosphérique, dans les conditions de l'équilibre thermodynamique ; le temps de séjour sur la résine du second lit est généralement de 20 à 60 minutes. De plus, à l'attaque du second lit catalytique, le rapport molaire acide/alcool est généralement excédentaire en acide.

Les Exemples suivants illustrent la présente invention sans toutefois en limiter la portée. Dans ces Exemples les pourcentages sont en poids sauf indication contraire et les abréviations suivantes ont été utilisées :
- AA : acide acrylique
- HQ : hydroquinone
- EMHQ : éther monométhylique de l'hydroquinone
- BuOH: butanol
- E2HOH : 2-éthylhexanol
- ABu : acrylate de butyle
- A2EH : acrylate de 2-éthylhexyle

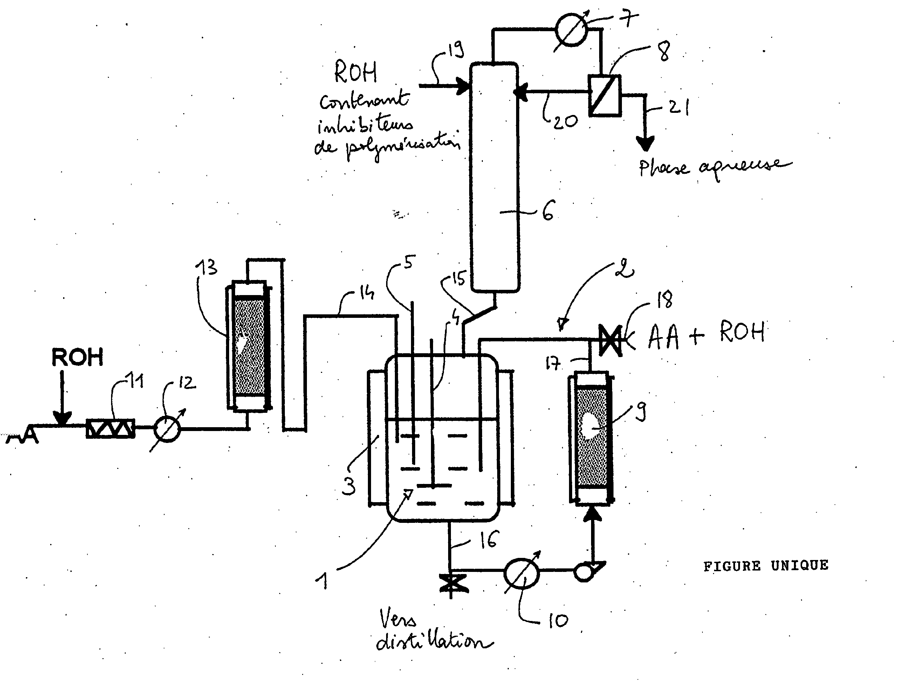

L'appareillage représenté sur la Figure unique du dessin annexé comprend une cuve de brassage 1 à laquelle est associée une boucle de circulation externe 2.

La cuve de brassage 1 est chauffée par une double enveloppe 3 alimentée par un thermofluide. Elle est agitée mécaniquement à l'aide d'un moyen d'agitation 4. Un bullage d'air nécessaire à la stabilisation du milieu réactionnel de la cuve 1 est effectué par une canne d'introduction 5.

La cuve 1 est surmontée par une colonne à distiller à garnissage 6 avec condenseur de tête 7, décanteur 8, recette et piège. Le réglage du niveau de l'interphase dans le décanteur s'effectue à l'aide d'une sonde de détection à haute impédance.

Sur la boucle de circulation 2, est placé un réacteur 9, à double enveloppe alimentée par un thermofluide et à garnissage de résine (première cartouche 9), et un échangeur de chaleur 10 destiné à abaisser la température du flux réactionnel avant passage sur la résine se trouvant dans la cartouche 9. La circulation des réactifs dans la cartouche 9 s'effectue dans le sens ascendant afin de maintenir la résine en suspension dans le liquide et favoriser ainsi les échanges de matière et de chaleur.

La cuve 1 est alimentée par le mélange des réactifs : acide acrylique et alcool, l'opération de mélange étant effectuée dans la mélangeur statique 11. Un préchauffeur 12, destiné à assurer la mise en température des réactifs, est placé à la sortie du mélangeur statique 11.

Dans une configuration préférée, telle qu'illustrée sur la Figure, un réacteur 13, à double enveloppe alimentée par un thermofluide et à garnissage de résine (seconde cartouche 13) est placé en amont de la cuve 1. Le mélange réactionnel y circule dans le sens ascendant et la résine est maintenue en suspension dans le liquide. Ce réacteur tubulaire catalytique 13, qui fonctionne dans les conditions de l'équilibre thermodynamique est en fait un premier étage de réaction qui assure une partie de la conversion des réactifs avant attaque du second étage de finition, lequel fonctionne en déplacement d'équilibre.

Le mélange des réactifs 14, préchauffé en 12 et ayant subi une conversion partielle dans le réacteur 13, est adressé à la cuve de brassage 1. L'eau de réaction est éliminée de la cuve 1 sous la forme d'un azéotrope 15. Le brut de réaction 16 est, à la sortie de la cuve 1, pour partie dirigé vers la distillation, pour partie vers la cartouche de résine 9. Le brut de réaction 17 est, à la sortie de la cartouche 9, renvoyé dans la cuve de brassage 1.

Les réactifs peuvent également être introduits directement en 18 sur la boucle de circulation 2.

Par ailleurs, une tuyauterie d'alimentation 19, en tête de colonne 6, permet d'alimenter cette dernière par de l'alcool contenant des inhibiteurs de polymérisation.

Le décanteur 8 en tête de colonne 6 permet de séparer une phase organique 20 qui est refluée sur la colonne 6 et une phase aqueuse 21 qui est éliminée.

Les résines utilisées dans les Exemples sont les suivantes :
Résine n°1 : Résine commercialisée par la Société "ROHM & HAAS" sous la dénomination "XE 586" ; ses caractéristiques sont les suivantes :
   - capacité ionique : 0,5 éq./l ;
   - diamètre moyen : 0,53 mm.
Résine n°2 : Résine commercialisée par la Société "MITSUBISHI CHEM." sous la dénomination "DIAION PK 208".

### EXEMPLE 1 : Fabrication d'acrylate de butyle

L'appareillage utilisé dans cet exemple est celui décrit avec référence à la Figure unique du dessin annexé, excepté que la cuve de brassage 1 n'est pas précédée par la cartouche de résine 13 à double enveloppe. La cuve 1 à double enveloppe thermostatée 3 est en acier inoxydable et a un volume utile de 700 ml. La double enveloppe 3 est alimentée par de l'huile à 130°C. L'agitateur 4 est de type ancre. La colonne à garnissage 6 a un diamètre intérieur de 22,5 mm et un décanteur de 50 ml de volume.

Le réacteur catalytique 9 est constitué d'un tube en acier inoxydable, à double enveloppe alimentée par de l'huile à 130°C ; son diamètre intérieur est de 30 mm et sa hauteur de 600 mm. Il contient 134 g de Résine n°1 préalablement séchée en étuve ventilée.

L'ensemble peut fonctionner sous dépression.

Les réactifs AA (+ inhibiteurs) et BuOH sont alimentés dans le rapport molaire AA/BuOH de 0,77/1 à un débit de 270 ml/h dans la cuve 1. Les inhibiteurs précités sont constitués par 500 ppm de 1,1,3-tris(2-méthyl-4-hydroxy-5-terbutylphényl)butane (TOPANOL® CA de la Société SEAL SANDS CHEMICALS) et 500 ppm d'HQ par rapport à l'AA. La température dans la cuve 1 est de 100°C et celle au coeur du lit de résine 9, de 92°C. Le brut de réaction 16 est recirculé par le fond de la cuve 1 à l'aide d'une pompe centrifuge à un débit de 4 l/h sur le lit de résine 9. Le taux de renouvellement du réacteur 1 est d'environ 10 fois/h.

La pression de service est de 3,95 x 10⁴ Pa (300 mm Hg), et la température de tête de la colonne 6 est de 75-76°C. L'eau de réaction est distillée sous la forme d'un azéotrope avec BuOH.

Le débit de la phase aqueuse 21 est de 23 ml/h, la composition massique de ladite phase aqueuse étant :
- Eau 9,3 %
- BuOH 6,9 %
- AA 200 ppm.

Le débit de sortie du brut réactionnel est de 246 ml/h, la composition massique de celui-ci étant
- BuOH 19,3 %
- AA 5,8 %
- Eau 0,25 %
- ABu 72,4 %
- Acétate de butyle 84 ppm
- Oxyde de butyle 2000 ppm
- Propionate de butyle 257 ppm
- Hydroxypropionate de butyle 352 ppm
- Butoxypropionate de butyle 900 ppm.

Le taux de conversion de l'AA est de 87 % ; la sélectivité par rapport à l'AA consommé est de 99,2 %.

### EXEMPLE 2 : Fabrication d'acrylate de 2-éthylhexyle

On utilise le même appareillage et la même résine qu'à l'Exemple 1.

Un mélange d'AA (81 g/h) et d'E2HOH (64 g/h) contenant 500 ppm de 1,1,3-tris(2-méthyl-4-hydroxy-5-terbutylphényl)butane (TOPANOL® CA) et 500 ppm d'HQ comptés par rapport à la charge globale AA + E2HOH, est introduit en continu dans la cuve de brassage 1. La température dans la cuve 1 est de 99°C.

Un complément d'E2HOH contenant 500 ppm d'HQ est effectué en tête de colonne 6 (96 g/h) afin d'éviter la montée de l'AA. Le rapport molaire global AA/E2HOH est ainsi de 0,91/1.

Le brut réactionnel est recirculé à un débit de 36 l/g sur la cartouche de résine 9. La température au coeur du lit de résine 9 est de 95°C.

L'eau de réaction est éliminée sous forme d'azéotrope Eau/E2HOH contenant, en outre, de l'AA et de l'A2EH.

Les vapeurs sont condensées en tête et décantées à température ambiante.

La pression en tête est de 1,86 x 10⁴ Pa (140 mm Hg).

La phase aqueuse de décantation est soutirée à un débit de 12,4 g/h. Elle contient 96 % d'eau et 3,7 % d' E2HOH.

La phase organique a la composition massique suivante
- AA 3 %
- E2HOH 95,7 %
- A2EH 0,4 %
- Octènes 830 ppm

Le brut de réaction est soutiré en continu à un débit de 226 g/h. Sa composition massique est la suivante :
- E2HOH 26 %
- AA 10,8 %
- Eau 0,9 %
- A2EH 60,5 %
- Octènes 450 ppm
- Dioctyléther 900 ppm
- Hydroxypropionate de 2-éthylhexyle 770 ppm
- Acryloxypropionate de 2-éthylhexyle 500 ppm
- 2-Ethylhexyl oxypropionate de 2-éthylhexyle 650 ppm.

Le taux de conversion de l'AA est de 69,4 %; la sélectivité par rapport à l'AA consommé est de 94,5 % ; et la sélectivité par rapport à l'E2HOH consommé est de 97 %.

### EXEMPLE 3 : Fabrication d'acrylate de 2-éthylhexyle

On utilise l'appareillage de l'Exemple 1, précédé du réacteur tubulaire 13 décrit avec référence avec la Figure unique du dessin annexé.

Le réacteur 13 est un réacteur tubulaire à double enveloppe en acier inoxydable alimentée par de l'huile à 130°C. Il contient 134 g de Résine n°1 séchée en étuve ventilée.

Les réactifs : AA contenant des inhibiteurs de polymérisation (500 ppm de 1,1,3-tris(2-méthyl-4-hydroxy-5-terbutylphényl)butane (TOPANOL® CA) et 500 ppm d'HQ) et E2HOH, sont mélangés et préchauffés à 90°C, puis introduits par pompe à un débit de 615 ml/h à la base du réacteur catalytique 13. Le rapport molaire initial AA/E2HOH est de 1,26.

La température d'entrée dans la cartouche 13 est de 90°C. La température de sortie de la cartouche 13 est de 95°C. La réaction est effectuée sans déplacement de l'équilibre. Le sens du passage des réactifs sur la résine est ascendant. Le temps de séjour sur la résine est de 36 min.

La composition massique du brut de sortie de la cartouche 13 est la suivante :
- E2HOH 29,6 %
- AA 24,7 %
- Eau 4,4 %
- A2EH 41,2 %
- Octènes 320 ppm
- Impuretés maléiques (acide maléique + mono- + dimaléates) 1300 ppm
- Hydroxypropionate de 2-éthylhexyle 0,25 %
- Acryloxypropionate de 2-éthylhexyle 0,3 %
- 2-Ethylhexyl oxypropionate de 2-éthylhexyle 0,2 %.

Le taux de conversion de l'AA est de 51,2 % (environ 98,5 % de l'équilibre thermodynamique).

Le brut de sortie de la cartouche 13 est stocké dans une capacité intermédiaire, puis expédié par pompe dans le réacteur 1.

Le mélange réactionnel est introduit à un débit de 188,1 g/h dans la cuve de brassage 1.

Un complément d'E2HOH (63 g/h) contenant 500 ppm d'HQ est effectué en tête de colonne, ce qui ramène le rapport molaire global (premier + second étage) AA/E2HOH à 0,8/1.

Un léger bullage d'air est introduit dans la cuve de brassage 1. La double enveloppe de la cuve est alimentée par de l'huile à 150°C. La température dans la cuve 1 est de 104-105°C. La température au coeur de la cartouche de résine est de 95°C. La pression en tête de colonne est de 6,66 x 10³ Pa (50 mm Hg). Le débit de recirculation est de 30 l/h.

Le brut de sortie du deuxième étage (233,8 g/h) a la composition massique suivante :
- E2HOH 27,5 %
- AA 6,8 %
- Eau 0,14 %
- A2EH 63,5 %
- Octènes 110 ppm
- Ethers 550 ppm
- Impuretés maléiques (acide maléique + mono- + dimaléates) 1500 ppm
- Dimère d'AA ppm 250 ppm
- Hydroxypropionate de 2-éthylhexyle 0,3 %
- Acryloxypropionate de 2-éthylhexyle 0,8 %
- 2-Ethylhexyl oxypropionate de 2-éthylhexyle 0,9 %.

Le taux de conversion global d'AA est de 79 %. La sélectivité par rapport à l'AA consommé est de 96 %. La sélectivité par rapport à l'E2HOH consommé est de 98,7 %.

L'eau de réaction est éliminée en continu sous forme d'un azéotrope Eau/E2HOH contenant en outre des octènes, de l'AA, de l'A2EH.

La phase aqueuse de décantation est éliminée ; la phase organique est refluée dans la colonne.

La phase organique a la composition massique suivante :
- Eau 4,1 %
- AA 16,7 %
- 2-Ethyl hexanol 79 %
- Octènes 140 ppm.

### EXEMPLE 4 : Fabrication d'acrylate de 2-éthylhexyle

On reprend l'Exemple 3 en remplaçant la Résine n°1 par la Résine n°2.

Le mélange de réactifs : AA (contenant 500 ppm de 1,1,3-tris(2-méthyl-4-hydroxy-5-terbutylphényl)butane (TOPANOL® CA) et 500 ppm d'HQ) et E2HOH est expédié par pompe à 90°C sur le premier étage de réaction 13 à un débit de 433 ml/h.

Le rapport molaire initial AA/E2HOH est de 1,25.

Le temps de séjour sur la résine 13 est de 27 min., et la température au coeur du lit de résine est de 95°C. La quantité de résine séchée en étuve ventilée mise en oeuvre est de 170 g.

La composition massique en sortie de la cartouche 13 est la suivante :
- E2HOH 30,3 %
- AA 24,2 %
- Eau 4,1 %
- A2EH 37,5 %
- Octènes 500 ppm
- Impuretés maléiques (Acide maléique + Mono- + Dimaléates) 1600 ppm (elles sont fonction de la qualité de l'AA technique utilisé)
- Hydroxypropionate de 2-éthylhexyle 1,2 %
- Acryloxypropionate de 2-éthylhexyle 1,3 %
- 2-Ethylhexyl oxypropionate de 2-éthylhexyle 0,2 %.

Le taux de conversion de l'AA est de 48,1%, soit 93,8% de l'équilibre thermodynamique.

Le brut de sortie du premier étage 13 est ensuite expédié par pompe vers le deuxième étage pour y être post estérifié.

Le débit d'alimentation est de 190,5 g/h. Un complément d'E2HOH contenant 500 ppm d'HQ est effectué en tête de colonne 6. Le rapport molaire global (premier + second étage) AA/E2HOH est de 0,8/1. Un léger bullage d'air est effectué dans la cuve de brassage 1. La température à l'intérieur de la cuve 1 est de 107°C. La température en tête de colonne 6 est de 38°C à la pression de 6,66 x 10³ Pa (50 mm Hg).

Le brut de sortie 1 est recirculé à un débit de 20,6 l/h sur la cartouche 9 garnie de 134 g de Résine sèche n°2.

La température au coeur du lit de résine 9 est de 96°C.

L'eau de réaction est éliminée sous forme d'un azéotrope en tête de colonne 6.

Les vapeurs sont condensées en tête de colonne et décantées à température ambiante.

La phase aqueuse est éliminée ; la phase organique est refluée sur la colonne.

Le brut en sortie deuxième étage a la composition massique suivante :
- E2HOH 37,4 %
- AA 10,7 %
- Eau 0,12 %
- A2EH 47,2 %
- Octènes 1000 ppm
- Ethers 500 ppm
- Impuretés maléiques (acide maléique + mono- + dimaléates) 1600 ppm
- Dimère d'AA 1000 ppm
- Hydroxypropionate de 2-éthylhexyle 1,3 %
- Acryloxypropionate de 2-éthylhexyle 2,3 %
- 2-Ethylhexyl oxypropionate de 2-éthylhexyle 0,8 %.

Le taux de conversion de l'AA est de 67%.

## Revendications

1. Procédé de fabrication d'un ester l'acide acrylique ou méthacrylique par estérification de l'acide acrylique ou méthacrylique par un alcool aliphatique, primaire ou secondaire, en C₁-C₁₂, en présence d'une résine cationique comme catalyseur, l'eau de réaction étant éliminée sous la forme d'un azéotrope avec l'alcool estérifiant ou avec un solvant, **caractérisé par le fait que** l'on conduit la réaction d'estérification en faisant passer de manière ascendante le mélange des réactifs à travers un lit (9) de ladite résine cationique, dans une boucle de recirculation (2) qui est associée à une cuve agitée (1) dans laquelle est assuré le mélange des réactifs et à partir de laquelle est assurée l'élimination azéotropique de l'eau de réaction.

2. Procédé selon la revendication 1, **caractérisé par le fait que** l'on conduit une réaction partielle en amont de la cuve agitée (1) en adressant le mélange des réactifs de manière ascendante à travers un second lit (13) de la résine cationique comme catalyseur.

3. Procédé selon l'une des revendications 1 et 2, **caractérisé par le fait que** l'alcool est choisi parmi le n-butanol, le 2-éthylhexanol et le n-octanol.

4. Procédé selon l'une des revendications 1 à 3, **caractérisé par le fait que** le rapport molaire global Acide Alcool est compris entre 0,6 et 1.

5. Procédé selon la revendication 4, **caractérisé par le fait que** le rapport molaire global Acide Alcool est compris entre 0,7 et 0,9.

6. Procédé selon l'une des revendications 1 à 5, **caractérisé par le fait que** l'on conduit la réaction en présence d'au moins un inhibiteur de polymérisation choisi notamment parmi la phénothiazine, l'hydroquinone, l'éther monométhylique de l'hydroquinone et les phénols encombrés, à des teneurs dans le milieu comprises entre 500 et 5000 ppm.

7. Procédé selon la revendication 6, **caractérisé par le fait que** le ou les inhibiteurs de polymérisation sont introduits avec les réactifs et en tête de la colonne à distiller (6) surmontant la cuve agitée (1). 9

8. Procédé selon l'une des revendications 1 à 7, **caractérisé par le fait que** l'on introduit une partie de l'alcool en tête de la colonne à distiller (6) surmontant la cuve agitée (1), un ou des inhibiteurs de polymérisation étant avantageusement associés à l'alcool introduit en tête de colonne (6), la proportion massique de l'alcool ainsi introduit en tête de colonne (6) par rapport à l'alcool introduit dans la cuve (1) étant de 20 à 60 %

9. Procédé selon la revendication 8, **caractérisé par le fait que** la proportion massique de l'alcool introduit en tête de colonne (6) par rapport à l'alcool introduit dans la cuve (1) est de 30 à 50 %.

10. Procédé selon l'une des revendications 1 à , **caractérisé par le fait que** la température en tête de lit de résine (9) est comprise entre 70 et 100°C ; la température de la cuve de brassage (1) est comprise entre 100 et 110°C, le mélange réactionnel sortant de la cuve (1) étant refroidi à 80-90°C, puis recirculé au travers du lit de résine (9).

11. Procédé selon la revendication 10, **caractérisé par le fait que** la température en tête de lit de résine est comprise entre 80 et 95°C.

12. Procédé selon l'une des revendications 1 à 11, **caractérisé par le fait que** la résine cationique est une résine cationique forte styrène/divinyl benzène à groupements sulfoniques ayant une capacité ionique comprise entre 0,5 et 2,2 équivalents/litre.

13. Procédé selon l'une des revendications 1 à 12, **caractérisé par le fait que** l'on fait circuler le mélange réactionnel à travers le lit de résine (9), le temps de séjour global sur le lit de résine (9) étant compris entre 0,5 et 2 h.

14. Procédé selon l'une des revendications 1 à 13, **caractérisé par le fait que** la pression de service en tête de la colonne à distiller (6) surmontant la cuve agitée (1) est de 9,33 x 10³ à 5,3 x 10⁴ Pa (70 à 400 mm Hg).

15. Procédé selon l'une des revendications 1 à 14, **caractérisé par le fait que** la température en tête du second lit (13) est de 80 à 95°C.

16. Procédé selon l'une des revendications 1 à 15, **caractérisé par le fait que** l'on fait fonctionner le réacteur contenant le second lit (13) à la pression atmosphérique, dans les conditions de l'équilibre thermodynamique.

17. Procédé selon l'une des revendications 1 à 16, **caractérisé par le fait que** le temps de séjour sur la résine du second lit (13) est de 20 à 60 minutes.

18. Procédé selon l'une des revendications 1 à 17, **caractérisé par le fait qu'**à l'attaque du second lit catalytique (13), le rapport molaire Acide/Alcool est excédentaire en acide.

## Claims

1. Process for preparing an acrylic or methacrylic ester by esterifying acrylic or methacrylic acid with a primary or secondary C₁-C₁₂ aliphatic alcohol in the presence of a cationic resin catalyst, the water of reaction being removed in the form of an azeotrope with the esterifying alcohol or with a solvent, **characterized in that** the esterification reaction is conducted by passing the mixture of reactants in upflow mode through a bed (9) of the said cationic resin in a recirculation loop (2) which is combined with a stirred tank (1) in which the reactants are blended and from which the water of reaction is removed azeotropically.

2. Process according to Claim 1, **characterized in that** a partial reaction is conducted upstream of the stirred tank (1) by passing the mixture of reactants in upflow mode through a second bed (13) of the cationic resin catalyst.

3. Process according to either of Claims 1 and 2, **characterized in that** the alcohol is selected from n-butanol, 2-ethylhexanol and n-octanol.

4. Process according to one of Claims 1 to 3, **characterized in that** the overall acid/alcohol molar ratio is between 0.6 and 1.

5. Process according to Claim 4, **characterized in that** the overall acid/alcohol molar ratio is between 0.7 and 0.9.

6. Process according to one of Claims 1 to 5, **characterized in that** the reaction is conducted in the presence of at least one polymerization inhibitor selected in particular from phenothiazine, hydroquinone, hydroquinone monomethyl ether, and sterically hindered phenols, at levels in the medium of between 500 and 5 000 ppm.

7. Process according to Claim 6, **characterized in that** the polymerization inhibitor or inhibitors is or are introduced with the reactants at the top of the distillation column (6) surmounting the stirred tank (1).

8. Process according to one of Claims 1 to 7, **characterized in that** a portion of the alcohol is introduced at the top of the distillation column (6) surmounting the stirred tank (1), one or more polymerization inhibitors being advantageously combined with the alcohol introduced at the top of column (6), and the proportion by mass of the alcohol thus introduced at the top of column (6) relative to the alcohol introduced into the tank (1) being from 20 to 60%.

9. Process according to Claim 8, **characterized in that** the proportion by mass of the alcohol introduced at the top of column (6) relative to the alcohol introduced into the tank (1) is from 30 to 50%.

10. Process according to one of Claims 1 to 5, **characterized in that** the temperature at the top of the bed of resin (9) is between 70 and 100°C; the temperature of the blending tank (1) is between 100 and 110°C, the reaction mixture exiting the tank (1) being cooled at 80-90°C and then recirculated through the bed of resin (9).

11. Process according to Claim 10, **characterized in that** the temperature at the top of the bed of resin (9) is between 80 and 95°C.

12. Process according to one of Claims 1 to 11, **characterized in that** the cationic resin is a strong cationic styrene/divinylbenzene resin containing sulphonic groups, having an ionic capacity of between 0.5 and 2.2 equivalents/litre.

13. Process according to one of Claims 1 to 12, **characterized in that** the reaction mixture is circulated through the bed of resin (9), the overall residence time on the bed of resin (9) being between 0.5 and 2 h.

14. Process according to one of Claims 1 to 13, **characterized in that** the working pressure at the top of the distillation column (6) surmounting the stirred tank (1) is from 9.33 x 10³ to 5.3 x 10⁴ Pa (from 70 to 400 mmHg).

15. Process according to one of Claims 1 to 14, **characterized in that** the temperature at the top of the second bed (13) is from 80 to 95°C.

16. Process according to one of Claims 1 to 15, **characterized in that** the reactor containing the second bed (13) is operated at atmospheric pressure under conditions of thermodynamic equilibrium.

17. Process according to one of Claims 1 to 16, **characterized in that** the residence time on the resin of the second bed (13) is from 20 to 60 minutes.

18. Process according to one of Claims 1 to 17, **characterized in that**, at the input to the second catalytic bed (13), the acid/alcohol molar ratio is top-heavy in acid.

## Patentansprüche

1. Verfahren zur Herstellung eines Acryl- oder Methacrylsäureesters durch Veresterung von Acryl- oder Methacrylsäure mit einem primären oder sekundären aliphatischen C₁-C₁₂-Alkohol in Gegenwart eines kationischen Harzes als Katalysator, wobei das Reaktionswasser in Form eines Azeotrops mit dem veresternden Alkohol oder mit einem Lösungsmittel entfernt wird, **dadurch gekennzeichnet, dass** die Veresterungsreaktion durchgeführt wird, indem man das Reagenziengemisch aufsteigend über ein Bett (9) aus dem kationischen Harz in eine Rezirkulationsschleife (2) leitet, die mit einem gerührten Behälter (1) verbunden ist, in dem das Mischen der Reagenzien gewährleistet wird und aus dem die azeotrope Entfernung des Reaktionswassers gewährleistet wird.

2. Verfahren nach Anspruch 1, **dadurch gekennzeichnet, dass** eine partielle Reaktion stromaufwärts des gerührten Behälters (1) durchgeführt wird, indem das Gemisch von Reagenzien aufsteigend über ein zweites Bett (13) aus dem kationischen Harz als Katalysator geleitet wird.

3. Verfahren nach einem der Ansprüche 1 und 2, **dadurch gekennzeichnet, dass** der Alkohol aus n-Butanol, 2-Ethylhexanol und n-Octanol ausgewählt ist.

4. Verfahren nach einem der Ansprüche 1 bis 3, **dadurch gekennzeichnet, dass** das Gesamtmolverhältnis Säure/Alkohol zwischen 0,6 und 1 beträgt.

5. Verfahren nach Anspruch 4, **dadurch gekennzeichnet, dass** das Gesamtmolverhältnis Säure/Alkohol zwischen 0,7 und 0,9 beträgt.

6. Verfahren nach einem der Ansprüche 1 bis 5, **dadurch gekennzeichnet, dass** die Reaktion in Gegenwart mindestens eines Polymerisationsinhibitors durchgeführt wird, der insbesondere aus Phenothiazin, Hydrochinon, Hydrochinonmonomethylether und raumgreifenden Phenolen mit Gehalten im Medium zwischen 500 und 5000 ppm ausgewählt ist.

7. Verfahren nach Anspruch 6, **dadurch gekennzeichnet, dass** der oder die Polymerisationsinhibitor(en) mit den Reagenzien und am Kopf der oberhalb des Rührbehälters (1) angebrachten Destillierkolonne (6) eingebracht wird (werden).

8. Verfahren nach einem der Ansprüche 1 bis 7, **dadurch gekennzeichnet, dass** ein Teil des Alkohols am Kopf der oberhalb des Rührbehälters (1) angebrachten Destillierkolonne (6) eingebracht wird, wobei vorteilhafterweise dem am Kopf der Kolonne (6) eingebrachten Alkohol ein oder mehrere Polymerisationsinhibitoren beigefügt werden, wobei der Massenanteil des derart am Kopf der Kolonne (6) eingebrachten Alkohols, bezogen auf den in den Behälter (1) eingebrachten Alkohol, 20 bis 60% beträgt.

9. Verfahren nach Anspruch 8, **dadurch gekennzeichnet, dass** der Massenanteil des am Kopf der Kolonne (6) eingebrachten Alkohols, bezogen auf den in den Behälter (1) eingebrachten Alkohol, 30 bis 50% beträgt.

10. Verfahren nach einem der Ansprüche 1 bis 9, **dadurch gekennzeichnet, dass** die Temperatur am Kopf des Harzbetts (9) zwischen 70 und 100°C beträgt, die Temperatur des Rührbehälters (1) zwischen 100 und 110°C beträgt, das aus dem Behälter (1) herauskommende Reaktionsgemisch auf 80-90°C abgekühlt und dann über das Harzbett (9) rezirkuliert wird.

11. Verfahren nach Anspruch 10, **dadurch gekennzeichnet, dass** die Temperatur am Kopf des Harzbetts zwischen 80 und 95°C beträgt.

12. Verfahren nach einem der Ansprüche 1 bis 11, **dadurch gekennzeichnet, dass** das kationische Harz ein stark kationisches Styrol/Divinylbenzol-Harz mit Sulfonsäuregruppen ist, das eine Ionenkapazität zwischen 0,5 und 2,2 Äquivalenten/Liter besitzt.

13. Verfahren nach einem der Ansprüche 1 bis 12, **dadurch gekennzeichnet, dass** das Reaktionsgemisch über das Harzbett (9) zirkuliert wird, wobei die Gesamtverweildauer auf dem Harzbett (9) zwischen 0,5 und 2 Std. beträgt.

14. Verfahren nach einem der Ansprüche 1 bis 13, **dadurch gekennzeichnet, dass** der Arbeitsdruck am Kopf der oberhalb des Rührbehälters (1) angebrachten Destillierkolonne (6) 9,33 x 10³ bis 5,3 x 10⁴ Pa (70 bis 400 mm Hg) beträgt.

15. Verfahren nach einem der Ansprüche 1 bis 14, **dadurch gekennzeichnet, dass** die Temperatur am Kopf des zweiten Betts (13) 80 bis 95°C beträgt.

16. Verfahren nach einem der Ansprüche 1 bis 15, **dadurch gekennzeichnet, dass** man den Reaktor, der das zweite Bett (13) enthält, bei Atmosphärendruck unter thermodynamischen Gleichgewichtsbedingungen arbeiten lässt.

17. Verfahren nach einem der Ansprüche 1 bis 16, **dadurch gekennzeichnet, dass** die Verweildauer auf dem Harz des zweiten Betts (13) 20 bis 60 Minuten beträgt.

18. Verfahren nach einem der Ansprüche 1 bis 17, **dadurch gekennzeichnet, dass** bei Einwirkung des zweiten katalytischen Betts (13) das Molverhältnis Säure/Alkohol einen Säureüberschuss aufweist.
